# EUROPEAN PATENT APPLICATION

(11) **EP 1 723 955 A1**
(43) Date of publication of application: **22.11.2006**
(21) Application number: 05719994.5
(22) Date of filing: 04.03.2005
(51) Int. Cl.: A61K 31/4164, A61K 31/495, A61K 31/551, A61P 9/10, C07D 243/08, C07D 295/08

(54) **MEDICINE FOR PREVENTION AND/OR TREATMENT OF ISCHEMIC CIRCULATORY DISEASE**

(30) Priority: 05.03.2004 JP 2004063167
(71) Applicant: Mitsubishi Pharma Corporation, Osaka-shi, Osaka 541-0046 (JP)
(72) Inventor: TATSUNO, Jun, c/o MITSUBISHI PHARMA CORPORATION, Chuo-ku, Tokyo 1038405 (JP)
(74) Representative: HOFFMANN EITLE
(86) International application number: PCT/JP2005/003722
(87) International publication number: WO 2005/084668

(57) **Abstract**

A medicament for preventing and/or treating an ischemic cardiovascular disease, which contains as an active ingredient a compound of an aminobenzenesulfonic acid derivative represented by the following general formula (I): 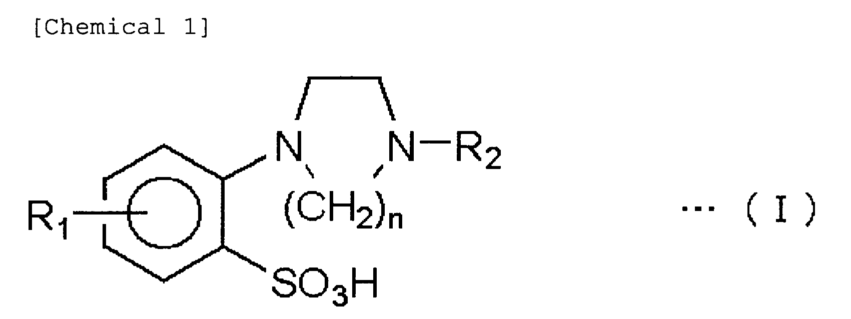 (refer to the description for the symbols in the formula) or a salt thereof, or a hydrate or solvate thereof, **characterized in that** it is administered to a patient undergoing percutaneous coronary intervention.

## Description

### Technical Field

The present invention relates to a medicament for preventing and/or treating an ischemic cardiovascular disease characterized in that it is administered to a patient undergoing percutaneous coronary intervention.

### Background Art

It is known that an aminobenzenesulfonic acid derivative represented by the following general formula (I): 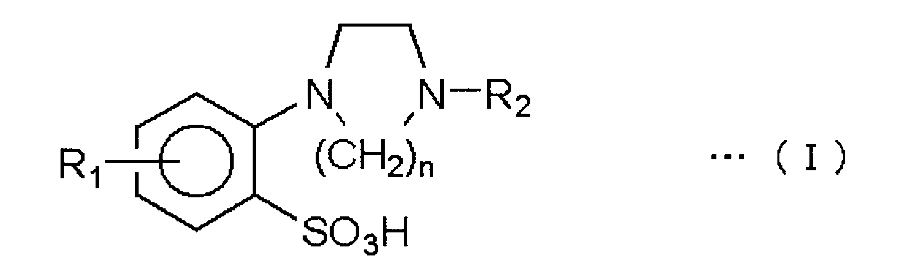 (wherein R₁ represents a hydrogen atom, a C₁-C₆ alkyl group, a C₃-C₇ cycloalkyl group, a halogenated C₁-C₄ alkyl group, a halogen atom or a C₆-C₁₂ aryl group; R₂ represents a hydrogen atom, a C₁-C₆ alkyl group or a C₇-C₁₂ aralkyl group which may have one or more substituents selected from the group consisting of a cyano group, a nitro group, a C₁-C₆ alkoxy group, a halogen atom, a C₁-C₆ alkyl group and an amino group; and n represents an integer of 1 to 4) or a salt thereof, or a hydrate or solvate thereof has suppressing action on hyper intracellular accumulation of calcium ion in myocardium cells or vessel smooth muscles (Patent Document 1: JP-A-3-7263, European Patent Application No. 390654). Further, in addition to this, such a compound is known to be effective in preventing or treating a heart disease (Patent Document 2: JP-A-4-139127), to be effective in preventing or treating cardiomyopathy (Patent Document 3: JP-A-10-298077), to be effective in preventing or treating dysfunction of diastolic of heart (Patent Document 4: WO 99/40919, European Patent Application No. 1062948), to be effective in preventing or treating a nervous system disorder (Patent Document 5: WO 01/45739), to be effective in preventing or treating heart failure or arrhythmia in a diabetic ischemic heart disease (Patent Document 6: WO 02/72097), and to inhibit the sodium/calcium exchange system (Patent Document 7: WO 03/9897).

However, it was not clear whether or not such a compound is effective in preventing and/or treating a patient with ischemic cardiovascular disease undergoing percutaneous coronary intervention in the present situation.
Patent Document 1: JP-A-3-7263, European Patent Application No. 390654
Patent Document 2: JP-A-4-139127
Patent Document 3: JP-A-10-298077
Patent Document 4: WO 99/40919, European Patent Application No. 1062948
Patent Document 5: WO 01/45739, European Patent Application No. 1249245
Patent Document 6: WO 02/72097, European Patent Application No. 1374868
Patent Document 7: WO 03/9897

### Disclosure of the invention

An object of the present invention is to provide a medicament for preventing and/or treating an ischemic cardiovascular disease characterized in that it is administered to a patient undergoing percutaneous coronary intervention.

The present inventors have conducted extensive studies to achieve the above-mentioned object, and as a result, they found that the administration of a specific aminobenzenesulfonic acid derivative or a salt thereof, or a hydrate or solvate thereof to a patient undergoing percutaneous coronary intervention can prevent myocardial injury and preserve myocardial function, and thus the present invention has been completed.

That is, the gists of the present invention are as follows.
1. A medicament for preventing and/or treating an ischemic cardiovascular disease, which comprises as an active ingredient a compound of an aminobenzenesulfonic acid derivative represented by the following general formula (I): 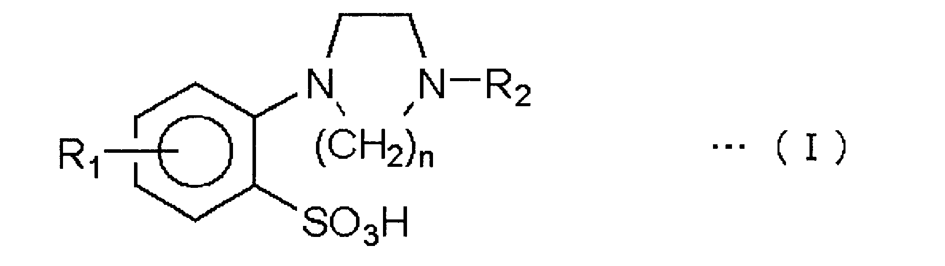 (wherein R₁ represents a hydrogen atom, a C₁-C₆ alkyl group, a C₃-C₇ cycloalkyl group, a halogenated C₁-C₄ alkyl group, a halogen atom or a C₆-C₁₂ aryl group; R₂ represents a hydrogen atom, a C₁-C₆ alkyl group or a C₇-C₁₂ aralkyl group which may have one or more substituents selected from the group consisting of a cyano group, a nitro group, a C₁-C₆ alkoxy group, a halogen atom, a C₁-C₆ alkyl group and an amino group; and n represents an integer of 1 to 4) or a salt thereof, or a hydrate or solvate thereof, characterized in that it is administered to a patient undergoing percutaneous coronary intervention.
2. The medicament according to 1, wherein the ischemic cardiovascular disease is myocardial infarction or angina pectoris.
3. The medicament according to 2, wherein the myocardial infarction is ST-segment elevation myocardial infarction.
4. The medicament according to 3, wherein the ST-segment elevation myocardial infarction is anterior ST-segment elevation myocardial infarction.
5. The medicament according to any one of 1 to 4, wherein the substitution position of R₁ is the 5-position.
6. The medicament according to any one of 1 to 5, wherein n is 2.
7. The medicament according to any one of 1 to 6, wherein R₂ is a hydrogen atom, a C₁-C₃ alkyl group or a C₇₋C₁₂ aralkyl group which may have one or more substituents selected from a C₁-C₃ alkyl group, a C₁-C₃ alkoxy group and a halogen atom.
8. The medicament according to any one of 1 to 7, wherein R₂ is a C₇-C₁₂ aralkyl group which may have one or more substituents selected from a hydrogen atom and a C₁-C₃ alkoxy group.
9. The medicament according to any one of 1 to 8, wherein R₂ is a hydrogen atom.
10. The medicament according to any one of 1 to 9, wherein R₁ is a hydrogen atom, a C₁-C₆ alkyl group, a C₅-C₆ cycloalkyl group, a trifluoromethyl group, a halogen atom or a phenyl group.
11. The medicament according to any one of 1 to 10, wherein R₁ is a C₁-C₃ alkyl group, a cyclohexyl group, a trifluoromethyl group, a chlorine atom, a bromine atom or a phenyl group.
12. The medicament according to any one of 1 to 11, wherein R₁ is a methyl group or a propyl group.
13. The medicament according to any one of 1 to 4, wherein the active ingredient is selected from the following compounds:
   5-methyl-2-(1-piperazinyl)benzenesulfonic acid;
   5-trifluoromethyl-2-(1-piperazinyl)benzenesulfonic acid;
   5-n-propyl-2-(1-piperazinyl)benzenesulfonic acid;
   5-phenyl-2-(1-piperazinyl)benzenesulfonic acid;
   5-chloro-2-(1-piperazinyl)benzenesulfonic acid;
   5-bromo-2-(1-piperazinyl)benzenesulfonic acid;
   5-iso-propyl-2-(1-piperazinyl)benzenesulfonic acid;
   5-cyclohexyl-2-(1-piperazinyl)benzenesulfonic acid;
   5-n-propyl-2-(1-homopiperazinyl)benzenesulfonic acid;
   5-n-propyl-2-[4-(2,3,4-trimethoxybenzyl)-1-piperazinyl]benzenesulfonic acid; and
   5-n-propyl-2-[4-(3,4-dimethoxybenzyl)-1-piperazinyl]benzenesulfonic acid.
14. The medicament according to Claim 13, wherein the active ingredient is selected from the following compounds:
   5-methyl-2-(1-piperazinyl)benzenesulfonic acid; and
   5-n-propyl-2-(1-piperazinyl)benzenesulfonic acid.
15. The medicament according to any one of 1 to 14, wherein the active ingredient is 5-methyl-2-(1-piperazinyl)benzenesulfonic acid monohydrate.
16. A medicament for preventing and/or treating anterior ST-segment elevation myocardial infarction, which comprises as an active ingredient 5-methyl-2-(1-piperazinyl)benzenesulfonic acid monohydrate, characterized in that it is administered to a patient undergoing percutaneous coronary intervention.
17. The medicament according to any one of 1 to 16, characterized in that the patient undergoing percutaneous coronary intervention has TIMI flow grade 0 or 1.
18. The medicament according to any one of 1 to 17, characterized in that administration of the active ingredient is initiated before percutaneous coronary intervention is performed.
19. The medicament according to any one of 1 to 18, characterized in that the active ingredient is continuously administered intravenously for 48 hours.
20. A method for preventing and/or treating an ischemic cardiovascular disease characterized by administering a medicament comprising as an active ingredient a compound of an aminobenzenesulfonic acid derivative represented by the following general formula (I) : 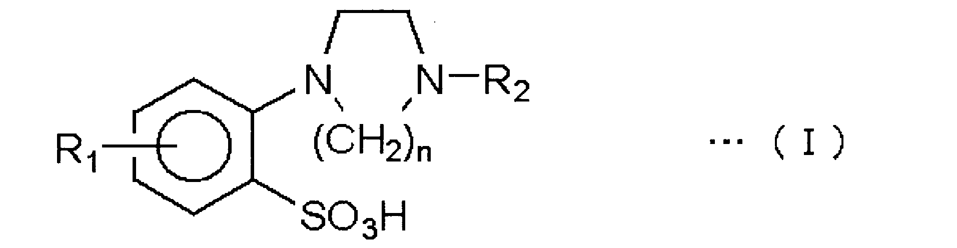 (wherein R₁ represents a hydrogen atom, a C₁-C₆ alkyl group, a C₃-C₇ cycloalkyl group, a halogenated C₁-C₄ alkyl group, a halogen atom or a C₆-C₁₂ aryl group; R₂ represents a hydrogen atom, a C₁-C₆ alkyl group or a C₇-C₁₂ aralkyl group which may have one or more substituents selected from the group consisting of a cyano group, a nitro group, a C₁-C₆ alkoxy group, a halogen atom, a C₁-C₆ alkyl group and an amino group; and n represents an integer of 1 to 4) or a salt thereof, or a hydrate or solvate thereof to a patient undergoing percutaneous coronary intervention.
21. The method according to 20, wherein the ischemic cardiovascular disease is myocardial infarction or angina pectoris.
22. The method according to 21, wherein the myocardial infarction is ST-segment elevation myocardial infarction.
23. The method according to 22, wherein the ST-segment elevation myocardial infarction is anterior ST-segment elevation myocardial infarction.
24. The method according to any one of 20 to 23, wherein the substitution position of R₁ is the 5-position.
25. The method according to any one of 20 to 24, wherein n is 2.
26. The method according to any one of 20 to 25, wherein R₂ is a hydrogen atom, a C₁-C₃ alkyl group or a C₇₋C₁₂ aralkyl group which may have one or more substituents selected from a C₁-C₃ alkyl group, a C₁-C₃ alkoxy group and a halogen atom.
27. The method according to any one of 20 to 26, wherein R₂ is a C₇-C₁₂ aralkyl group which may have one or more substituents selected from a hydrogen atom and a C₁-C₃ alkoxy group.
28. The method according to any one of 20 to 27, wherein R₂ is a hydrogen atom.
29. The method according to any one of 20 to 28, wherein R₁ is a hydrogen atom, a C₁-C₆ alkyl group, a C₅-C₆ cycloalkyl group, a trifluoromethyl group, a halogen atom or a phenyl group.
30. The method according to any one of 20 to 29, wherein R₁ is a C₁-C₃ alkyl group, a cyclohexyl group, a trifluoromethyl group, a chlorine atom, a bromine atom or a phenyl group.
31. The method according to any one of 20 to 30, wherein R₁ is a methyl group or a propyl group.
32. The method according to any one of 20 to 23, wherein the active ingredient is selected from the following compounds:
   5-methyl-2-(1-piperazinyl)benzenesulfonic acid;
   5-trifluoromethyl-2-(1-piperazinyl)benzenesulfonic acid;
   5-n-propyl-2-(1-piperazinyl)benzenesulfonic acid;
   5-phenyl-2-(1-piperazinyl)benzenesulfonic acid;
   5-chloro-2-(1-piperazinyl)benzenesulfonic acid;
   5-bromo-2-(1-piperazinyl)benzenesulfonic acid;
   5-iso-propyl-2-(1-piperazinyl)benzenesulfonic acid;
   5-cyclohexyl-2-(1-piperazinyl)benzenesulfonic acid;
   5-n-propyl-2-(1-homopiperazinyl)benzenesulfonic acid;
   5-n-propyl-2-[4-(2,3,4-trimethoxybenzyl)-1-piperazinyl]benzenesulfonic acid; and
   5-n-propyl-2-[4-(3,4-dimethoxybenzyl)-1-piperazinyl]benzenesulfonic acid.
33. The method according to 32, wherein the active ingredient is selected from the following compounds:
   5-methyl-2-(1-piperazinyl)benzenesulfonic acid; and
   5-n-propyl-2-(1-piperazinyl)benzenesulfonic acid.
34. The method according to any one of 20 to 33, wherein the active ingredient is 5-methyl-2-(1-piperazinyl)benzenesulfonic acid monohydrate.
35. A method for preventing and/or treating anterior ST-segment elevation myocardial infarction characterized by administering a medicament comprising as an active ingredient 5-methyl-2-(1-piperazinyl)benzenesulfonic acid monohydrate to a patient undergoing percutaneous coronary intervention.
36. The method according to any one of 20 to 35, wherein the patient undergoing percutaneous coronary intervention has TIMI flow grade 0 or 1.
37. The method according to any one of 20 to 36, characterized in that administration of the active ingredient is initiated before percutaneous coronary intervention is performed.
38. The medicament according to any one of 20 to 37, characterized in that the active ingredient is continuously administered intravenously for 48 hours.

### Best Mode for Embodying the Invention

In the present invention, an ischemic cardiovascular disease is a cardiovascular disease, which arises from the vessel occlusion due to any cause such as thrombus formation, and specifically, myocardial infarction or angina pectoris can be exemplified. In the present invention, as a preferred embodiment of the myocardial infarction, ST-segment elevation myocardial infarction (STEMI) can be exemplified, and as a more preferred embodiment, anterior ST-segment elevation myocardial infarction can be exemplified.

As a specific example of percutaneous coronary intervention (PCI), percutaneous transluminal coronary angioplasty (PTCA) and the like can be exemplified.

As the active ingredient of the medicament of the present invention, an aminobenzenesulfonic acid derivative represented by the above-mentioned general formula (I) or a salt thereof, or a hydrate or solvate thereof can be exemplified. Examples of the C₁-C₆ alkyl group defined for R₁ in the above-mentioned general formula (I) include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, an isohexyl group and the like. Examples of the C₃-C₇ cycloalkyl group include a cyclopropyl group, a cyclobutyl group, a cyclopentyl group, a cyclohexyl group, a cycloheptyl group and the like. Examples of the halogenated C₁-C₄ alkyl group include a trifluoromethyl group, a trifluoroethyl group, a pentafluoroethyl group and the like. Examples of the halogen atom include a fluorine atom, a chlorine atom, a bromine atom and the like. Examples of the C₆-C₁₂ aryl group include a phenyl group, a naphthyl group and the like.

Preferred examples of R₁ include a hydrogen atom, a C₁-C₆ alkyl group, a C₅-C₆ cycloalkyl group, a trifluoromethyl group, a halogen atom and a phenyl group, and more preferred examples of R₁ include a C₁-C₃ alkyl group, a cyclohexyl group, a trifluoromethyl group, a chlorine atom, a bromine atom and a phenyl group. R₁ is particularly preferably a methyl group or a propyl group.

As the C₁-C₆ alkyl group defined for R₂, for example, an alkyl group such as those defined for R₁ described above. Examples of the C₇-C₁₂ aralkyl group include a benzyl group, a phenethyl group, a naphthylmethyl group and the like. The aralkyl group may have one or more substituents selected from the group consisting of a cyano group; a nitro group; a C₁-C₆ alkoxy group such as a methoxy group, an ethoxy group, a propoxy group, an isopropoxy group, a butoxy group, an isobutoxy group, a tert-butoxy group, a pentyloxy group, an isopentyloxy group, a tert-pentyloxy group or a hexyloxy group; a halogen atom such as those defined for R₁ described above; an alkyl group such as those defined for R₁ described above; and an amino group.

Preferred examples of R₂ include a hydrogen atom, a C₁-C₃ alkyl group, and a C₇-C₁₂ aralkyl group which may have one or more substituents selected from a C₁-C₃ alkyl group, a C₁-C₃ alkoxy group and a halogen atom, and more preferred examples of R₂ include a hydrogen atom and a C₇-C₁₂ aralkyl group which may have one or more substituents selected from a C₁-C₃ alkoxy group. R₂ is particularly preferably a hydrogen atom.

Further, in the above-mentioned general formula (I), n is preferably 2.

Incidentally, preferred specific examples in the present invention include compounds shown in the following Table 1 and Table 2.

### Table 1

**Table 1**

| | | | | |
|---|---|---|---|---|
| 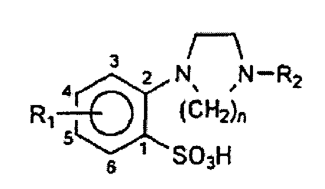 | | | | |

| Compound No. | Substitution position of R₁ | R₁ | n | R₂ |
|---|---|---|---|---|
| 1 | - | H | 2 | H |
| 2 | - 3 | -CH₃ | 2 | H |
| 3 | 3 | -CH₂CH₃ | 2 | H |
| 4 | 3 | -CH₂CH₂CH₃ | 2 | H |
| 5 | 3 | -CH(CH₃)₂ | 2 | H |
| 6 | 3 | -(CH₂)₃CH₃ | 2 | H |
| 7 | 4 | -CH₃ | 2 | H |
| 8 | 4 | -CH₂CH₃ | 2 | H |
| 9 | 4 | -(CH₂)₂CH₃ | 2 | H |
| 10 | 4 | -CH(CH₃)₂ | 2 | H |
| 11 | 4 | -(CH₂)₃CH₃ | 2 | H |
| 12 | 5 | -CH₃ | 2 | H |
| 13 | 5 | -CH₂CH₃ | 2 | H |
| 14 | 5 | -(CH₂)₂CH₃ | 2 | H |
| 15 | 5 | -CH(CH₃)₂ | 2 | H |
| 16 | 5 | -(CH₂)₃CH₃ | 2 | H |
| 17 | 5 | -(CH₂)₄CH₃ | 2 | H |
| 18 | 5 | -(CH₂)₅CH₃ | 2 | H |
| 19 | 6 | -CH₃ | 2 | H |
| 20 | 6 | -CH₂CH₃ | 2 | H |
| 21 | 6 | -(CH₂)₂CH₃ | 2 | H |
| 22 | - | H | 2 | -CH₃ |
| 23 | 3 | -CH₂CH₃ | 2 | -CH₃ |
| 24 | 3 | -(CH₂)₂CH₃ | 2 | -CH₃ |
| 25 | 3 | -CH(CH₃)₂ | 2 | -CH₃ |
| 26 | 3 | -(CH₂)₃CH₃ | 2 | -CH₃ |
| 27 | 4 | -CH₃ | 2 | -CH₃ |
| 28 | 4 | -CH₂CH₃ | 2 | -CH₃ |
| 29 | 4 | -(CH₂)₂CH₃ | 2 | -CH₃ |
| 36 | 5 | -CH₃ | 2 | -CH₃ |
| 31 | 5 | -CH₂CH₃ | 2 | -CH₃ |
| 32 | 5 | -(CH₂)₂CH₃ | 2 | -CH₃ |
| 33 | 5 | -CH(CH₃)₂ | 2 | -CH₃ |
| 34 | 5 | -(CH₂)₃CH₃ | 2 | -CH₃ |
| 35 | 5 | -(CH₂)₄CH₃ | 2 | -CH₃ |
| 36 | 5 | -(CH₂)₅CH₃ | 2 | -CH₃ |
| 37 | 6 | -CH₃ | 2 | -CH₃ |
| 38 | 6 | -CH₂CH₃ | 2 | -CH₃ |
| 39 | 6 | -(CH₂)₂CH₃ | 2 | -CH₃ |
| 40 | 6 | -CH(CH₃)₂ | 2 | -CH₃ |
| 41 | 6 | -(CH₂)₃CH₃ | 2 | -CH₃ |
| 42 | 3 | -(CH₂)₂CH₃ | 2 | -(CH₂)₂CH₃ |
| 43 | 4 | -(CH₂)₂CH₃ | 2 | -(CH₂)₂CH₃ |
| 44 | 5 | -CH₃ | 2 | -(CH₂)₂CH₃ |
| 45 | 5 | -CH₂CH₃ | 2 | -(CH₂)₂CH₃ |
| 46 | 5 | -(CH₂)₂CH₃ | 2 | -(CH₂)₂CH₃ |
| 47 | 5 | -CH(CH₃)₂ | 2 | -(CH₂)₂CH₃ |
| 48 | 5 | -(CH₂)₃CH₃ | 2 | -(CH₂)₂CH₃ |
| 49 | 5 | -(CH₂)₅CH₃ | 2 | -(CH₂)₂CH₃ |
| 50 | - | H | 2 | -(CH₂)₂CH₃ |
| 51 | - | H | 2 | 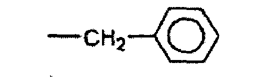 |
| 52 | 3 | -CH₃ | 2 | 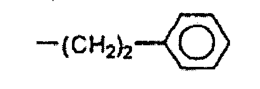 |
| 53 | 3 | -(CH₂)₂CH₃ | 2 | 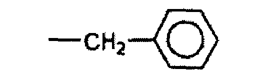 |
| 54 | 4 | -CH₃ | 2 | 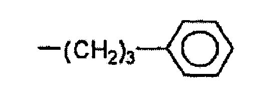 |
| 55 | 4 | -(CH₂)₂CH₃ | 2 | 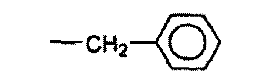 |
| 56 | 5 | -CH₃ | 2 | 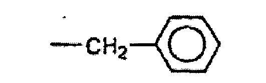 |
| 57 | 5 | -CH₂CH₃ | 2 | 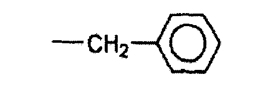 |
| 58 | 5 | -(CH₂)₂CH₃ | 2 | 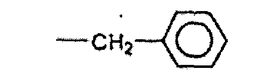 |
| 59 | 5 | -CH(CH₃)₂ | 2 | 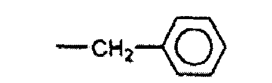 |
| 60 | 5 | -(CH₂)₃CH₃ | 2 | 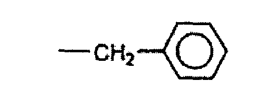 |
| 61 | 5 | -(CH₂)₄CH₃ | 2 | 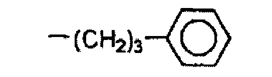 |
| 62 | 5 | -(CH₂)₂CH₃ | 2 | 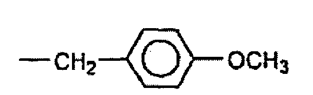 |
| 63 | 5 | -CH(CH₃)₂ | 2 | 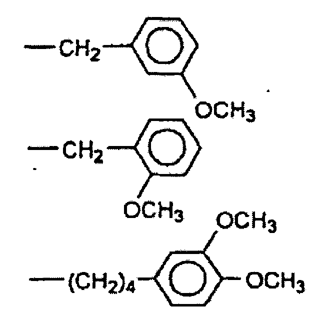 |
| 64 | 5 | -CH(CH₃)₂ | 2 | |
| 65 | 4 | -(CH₂)₂CH₃ | 2 | |
| 66 | 5 | -(CH₂)₂CH₃ | 2 | 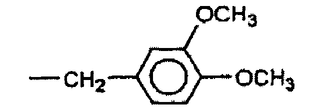 |
| 67 | 5 | -CH(CH₃)₂ | 2 | 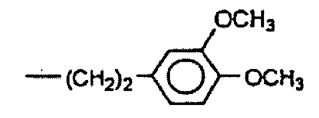 |
| 68 | 6 | -(CH₂)₂CH₃ | 2 | 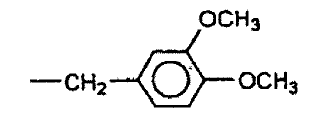 |
| 69 | 5 | -(CH₂)₂CH₃ | 2 | 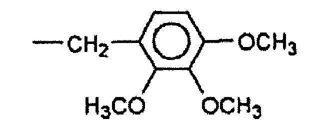 |
| 70 | 6 | -(CH₂)₂CH₃ | 2 | 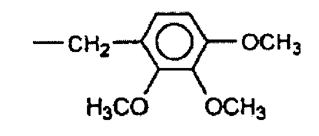 |
| 71 | 3 | -(CH₂)₂CH₃ | 2 | 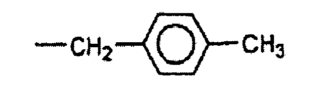 |
| 72 | 4 | -(CH₂)₂CH₃ | 2 | 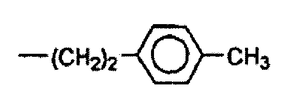 |
| 73 | 5 | -CCH₄)₂CH₃ | 2 | 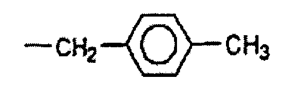 |
| 74 | 6 | -CH(CH₃)₂ | 2 | 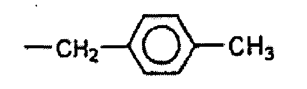 |
| 75 | 3 | -(CH₂)₂CH₃ | 2 | 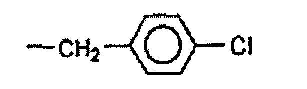 |
| 76 | 4 | -(CH₂)₂CH₃ | 2 | 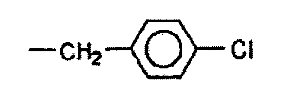 |
| 77 | 5 | -(CH₂)₂CH₃ | 2 | 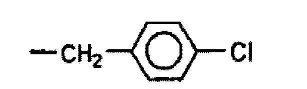 |
| 78 | 6 | -(CH₂)₂CH₃ | 2 | 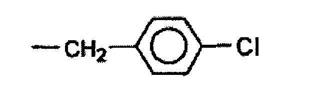 |
| 79 | 3 | -(CH₂)₂CH₃ | 2 | 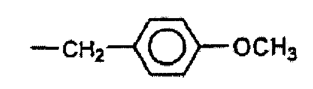 |
| 80 | 4 | -(CH₂)₂CH₃ | 2 | 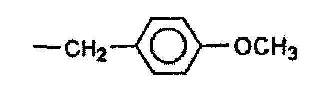 |
| 81 | 5 | -(CH₂)₂CH₃ | 2 | 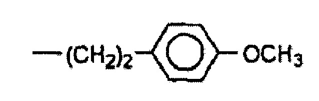 |
| 82 | 6 | -(CH₂)₂CH₃ | 2 | 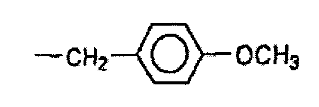 |
| 83 | - | H | 3 | H |
| 84 | 5 | -CH₃ | 3 | H |
| 85 | 5 | -CH₂CH₃ | 3 | H |
| 86 | 5 | -(CH₂)₂CH₃ | 3 | H |
| 87 | 5 | -CH(CH₃)₂ | 3 | H |
| 88 | 5 | -(CH₂)₂CH₃ | 3 | H |
| 89 | 5 | -(CH₂)₂CH₃ | 3 | -CH₃ |
| 90 | 5 | -(CH₂)₂CH₃ | 3 | 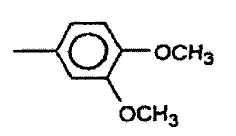 |
| 91 | 5 | 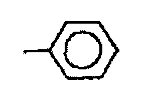 | 2 | H |
| 92 | 5 | -F | 2 | H |
| 93 | 5 | -Cl | 2 | H |
| 94 | 5 | -Br | 2 | H |
| 95 | 5 | -CF₃ | 2 | H |
| 96 | 5 | 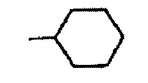 | 2 | H |
| 97 | 5 | 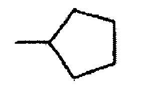 | 2 | H |
| 98 | 5 | 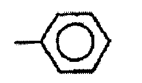 | 2 | -CH₃ |
| 99 | 5 | -CI | 2 | -CH₃ |
| 100 | 5 | -Br | 2 | -CH₃ |
| 101 | 5 | -CF₃ | 2 | -CH₃ |
| 102 | 5 | 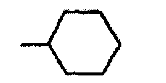 | 2 | -CH₃ |
| 103 | 5 | 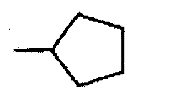 | 2 | -CH₃ |
| 104 | 5 | 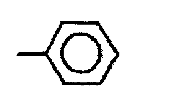 | 2 | 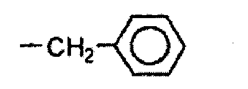 |
| 105 | 5 | -Cl | 2 | 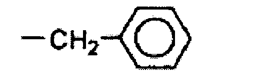 |
| 106 | 5 | -Br | 2 | 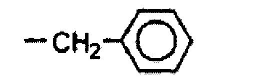 |
| 107 | 5 | -CF₃ | 2 | 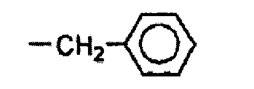 |
| 108 | 5 | 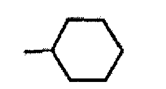 | 2 | 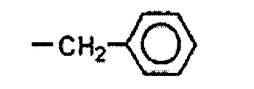 |
| 109 | 5 | 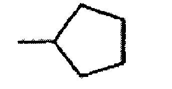 | 2 | 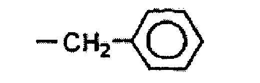 |

**Table 2**

| | | | | |
|---|---|---|---|---|
| 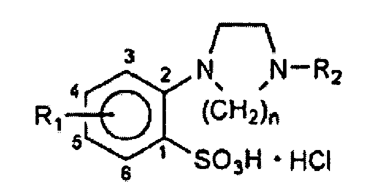 | | | | |

| Compound No. | Substitution position of R₁ | R₁ | **n** | **R**_{**2**} |
|---|---|---|---|---|
| 110 | 5 | -CH₂CH₂CH₃ | 2 | H |
| 111 | 5 | -CH(CH₃)₂ | 2 | H |
| 112 | 5 | 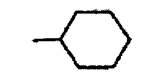 | 2 | H |
| 113 | 5 | 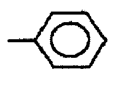 | **2** | **H** |
| 114 | 5 | -Cl | 2 | H |
| 115 | 5 | -Br | 2 | H |
| 116 | 5 | -CF₃ | 2 | H |

In Table 1 and Table 2 described above, a compound in which the substitution position of R₁ is the 5-position is preferred, and as a more preferred compound, the following compounds can be exemplified:
5-methyl-2-(1-piperazinyl)benzenesulfonic acid;
5-trifluoromethyl-2-(1-piperazinyl)benzenesulfonic acid;
5-n-propyl-2-(1-piperazinyl)benzenesulfonic acid;
5-phenyl-2-(1-piperazinyl)benzenesulfonic acid;
5-chloro-2-(1-piperazinyl)benzenesulfonic acid;
5-bromo-2-(1-piperazinyl)benzenesulfonic acid;
5-iso-propyl-2-(1-piperazinyl)benzenesulfonic acid;
5-cyclohexyl-2-(1-piperazinyl)benzenesulfonic acid;
5-n-propyl-2-(1-homopiperazinyl)benzenesulfonic acid;
5-n-propyl-2-[4-(2,3,4-trimethoxybenzyl)-1-piperazinyl]benzenesulfonic acid; and
5-n-propyl-2-[4-(3,4-dimethoxybenzyl)-1-piperazinyl]benzenesulfonic acid.

Incidentally, among the above-mentioned compounds, particularly preferable examples include 5-methyl-2- (1-piperazinyl)benzenesulfonic acid and 5-n-propyl-2- (1-piperazinyl)benzenesulfonic acid.

Further, pharmaceutically acceptable salts of the above-mentioned compounds are also included in the scope of the present invention. Examples of the salts of the above-mentioned compounds include alkali metal salts and alkaline earth metal salts such as sodium salts, potassium salts, magnesium salts, calcium salts and aluminum salts; amine salts (ammonium salts; lower alkylamine salts such as triethylamine salts; hydroxy-lower alkylamine salts such as 2-hydroxyethylamine salts, bis-(2-hydroxyethyl) amine salts, tris(hydroxymethyl)aminomethane salts, and N-methyl-D-glucamine salts; cycloalkylamine salts such as dicyclohexylamine salts; benzylamine salts such as N,N-dibenzylethylenediamine salts; dibenzylamine salts and the like); inorganic acid salts such as hydrochloric acid salts, hydrobromic acid salts, sulfuric acid salts, and phosphoric acid salts; organic acid salts such as fumaric acid salts, succinic acid salts, oxalic acid salts, and lactic acid salts and the like.

In addition to the compounds in free form or in the form of salts, any hydrates or solvates thereof can also be used as an active ingredient of the medicament of the present invention. Examples of a solvent which can form the solvates of the above-mentioned compound include methanol, ethanol, isopropyl alcohol, acetone, ethyl acetate, methylene chloride and the like.

As the active ingredient of the present invention, 5-methyl-2-(1-piperazinyl)benzenesulfonic acid monohydrate can be exemplified as the most preferred one.

The aminobenzenesulfonic acid derivative represented by the above-mentioned general formula (I) is a known compound, and can be readily synthesized according to the methods described in JP-A-3-7263 and JP-A-9-221479, European Unexamined Patent Publication Nos. 390654 and 779283, US Patent Nos. 5053409 and 5990113 and the like, and is a compound that can be readily obtained by those skilled in the art.

The medicament of the present invention can be applied orally or parenterally according to a standard method. Examples of the dosage form for oral administration include granules, fine granules, powders, tablets, hard capsules, soft capsules, syrups, emulsions, suspensions, solutions and the like. Examples of the dosage form for parenteral administration include injections, suppositories, transdermal preparations and the like.

The active ingredient of the present invention is contained together with a solid or liquid pharmaceutical carrier or a pharmaceutical additive which is used commonly such as an excipient, a stabilizer, a lubricant, a sweetener, a preservative or a suspending agent in the above-mentioned dosage form, and the content ratio of the preventive and/or therapeutic active ingredient to the carrier component is preferably in the range from 1% by weight to 90% by weight.

Examples of the solid component to be used include lactose, kaolin, sucrose, crystalline cellulose, cornstarch, talc, agar, pectin, acacia, stearic acid, magnesium stearate, lecithin, sodium chloride and the like. Examples of the liquid carrier include syrup, glycerol, peanut oil, polyvinylpyrrolidone, olive oil, ethanol, benzyl alcohol, propylene glycol, water and the like.

The dose of a substance to be used as the active ingredient can be suitably determined depending on, for example, the purpose of prevention or treatment, the kind of disease to be prevented or treated, the symptoms, body weight, age, and sexuality of a patient for each active ingredient. In general, a dose of about 0.001 mg to 100 mg per day can be administered parenterally to an adult, and a dose of about 0.01 mg to 1000 mg per day can be administered orally to an adult. It is preferred that the above-mentioned dose is administered once a day or several times a day as divided portions.

As a preferred administration method and administration period in the case of using 5-methyl-2-(1-piperazinyl)benzenesulfonic acid, continuous intravenous administration for 48 hours (2 days) is preferred.

In the present invention, TIMI flow grade refers to a grade classified according to the Thrombolysis in Myocardial Infraction trial (N Engl J Med 33:523, 1984), and a state where a contrast agent is not distally perfused beyond a culprit lesion is defined as TIMI flow grade 0, and a state where a small amount of a contrast agent is perfused beyond a culprit lesion, however it does not reach a distal vasculature is defined as TIMI flow grade 1.

In the present invention, the term "prevention" refers to administration of a medicament to a healthy subject who does not have any disease such as administration of a medicament for the purpose of preventing the occurrence of any disease. The term "treatment" refers to administration of a medicament to a patient diagnosed with a disease by a physician such as administration of a medicament for the purpose of alleviating the disease or the symptoms, or restoring the health. In addition, even if the purpose of the administration is to prevent the deterioration of the disease or the symptoms or to prevent an attack, in the case where the person to whom a medicament is administered is a patient who has been once diagnosed with some disease, it is categorized as treatment. Here, to a patient diagnosed with a disease, an intervention of administering a medicament for the purpose of alleviating the disease or the symptoms is categorized as treatment, however, an intervention of administering a medicament for the purpose of preventing the occurrence of another disease that has not occurred yet is categorized as prevention.

### Example

Hereinafter, the present invention will be described in more detail with reference to Example, however, the present invention is not limited to the following Example as long as it is not beyond the gists of the present invention.

A compound of the present invention referred to in the following Example is 5-methyl-2-(1-piperazinyl) benzenesulfonic acid monohydrate (hereinafter sometimes referred to as "caldaret"), and a compound prepared according to the method described in Example 1 in JP-A-9-221479 was used.

### (Example 1)

Three hundred and eighty-seven patients with ΣST elevation of 10 mm or more undergoing PCI were divided into 3 groups: a caldaret non-administration group; a 61.5 mg caldaret administration group (57.5 mg in terms of 5-methyl-2-(1-piperazinyl)benzenesulfonic acid) (low-dose group); and a 184.6 mg caldaret administration group (172.5 mg in terms of 5-methyl-2-(1-piperazinyl)benzenesulfonic acid) (high-dose group). To these patients, administration of placebo (the caldaret non-administration group) or caldaret (the low-dose group and the high-dose group) was initiated about 30 minutes before PCI was performed. Placebo or caldaret was continuously administered intravenously for 48 hours. At day 7 after administration (the initiation day of administration = day 0 after administration, and hereinafter the same), the infarct size, the left ventricular end-systolic volume (ESV), the left ventricular end-diastolic volume (EDV) and the left ventricular ejection fraction (LVEF) were measured by SPECT and the cardiac serum markers were measured. Incidentally, as for the serum markers, AUC of TnT at days 0 to 5 after administration and AUC of CK at days 0 to 3 after administration were measured.

Hereinafter, the measurement results in 247 patients with TIMI flow grade 0 or 1 diagnosed with anterior ST-segment elevation myocardial infarction (88 patients in the low-dose group, 70 patients in the high-dose group and 89 patients in the caldaret non-administration group) are shown.

| | Infarct size (% of the left ventricle) | ESV (ml) | EDV (ml) | LVEF (%) | Serum marker | |
|---|---|---|---|---|---|---|
| | | | | | TnT (mg/l) | CK (IU/I) |
| Low-dose administration group | 24.4 (a) | 80.7 (*) | 138.4 (*) | 36.6 | 5 | 1144 (*) |
| High-dose administration group | 25.7 (b) | 78.0 (*) | 134.1 (*) | 38.1 (*) | 4.3 (*) | 1157 (*) |
| Non-administration group | 30 | 106.1 | 166.9 | 31.6 | 5.7 | 1603 |

| | | | | | | |
|---|---|---|---|---|---|---|
| (*): p < 0.005, (a): p = 0.052, (b): p = 0.144 vs. non-administration group | | | | | | |

Further, the measurement results in 359 patients diagnosed with anterior ST-segment elevation myocardial infarction also show regardless of the TIMI flow grade that the caldaret administration groups (the low-dose group and the high-dose group) showed an improving tendency in all the measurement parameters compared with the non-administration group in the same manner as above.

From the above results, it was shown that caldaret can alleviate myocardial injury and preserve myocardial function of a patient with anterior ST-segment elevation myocardial infarction undergoing percutaneous coronary intervention.

### Industrial Applicability

It was found that the aminobenzenesulfonic acid derivative or a salt thereof, or a hydrate or solvate thereof of the present invention can alleviate myocardial injury and preserve myocardial function of a patient with anterior ST-segment elevation myocardial infarction undergoing percutaneous coronary intervention. Therefore, the above-mentioned aminobenzenesulfonic acid derivative or a salt thereof, or a hydrate or solvate thereof is useful as a medicament for preventing and/or treating an ischemic cardiovascular disease characterized in that it is administered to a patient undergoing percutaneous coronary intervention.

The present application is based on Japanese Patent Application No. 2004-63167 filed on March 5,2004, the contents of which are incorporated herein by reference in its entirety.

## Claims

1. A medicament for preventing and/or treating an ischemic cardiovascular disease, which comprises as an active ingredient a compound of an aminobenzenesulfonic acid derivative represented by the following general formula (I): 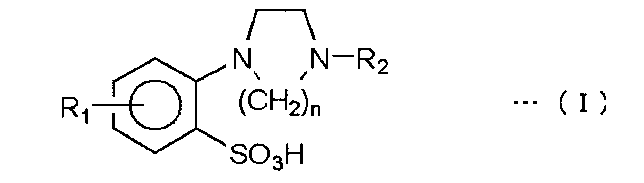 (wherein R₁ represents a hydrogen atom, a C₁-C₆ alkyl group, a C₃-C₇ cycloalkyl group, a halogenated C₁-C₄ alkyl group, a halogen atom or a C₆-C₁₂ aryl group; R₂ represents a hydrogen atom, a C₁-C₆ alkyl group or a C₇-C₁₂ aralkyl group which may have one or more substituents selected from the group consisting of a cyano group, a nitro group, a C₁-C₆ alkoxy group, a halogen atom, a C₁-C₆ alkyl group and an amino group; and n represents an integer of 1 to 4) or a salt thereof, or a hydrate or solvate thereof, **characterized in that** it is administered to a patient undergoing percutaneous coronary intervention.

2. The medicament according to Claim 1, wherein the ischemic cardiovascular disease is myocardial infarction or angina pectoris.

3. The medicament according to Claim 2, wherein the myocardial infarction is ST-segment elevation myocardial infarction.

4. The medicament according to Claim 3, wherein the ST-segment elevation myocardial infarction is anterior ST-segment elevation myocardial infarction.

5. The medicament according to any one of Claims 1 to 4, wherein the substitution position of R₁ is the 5-position.

6. The medicament according to any one of Claims 1 to 5, wherein n is 2.

7. The medicament according to any one of Claims 1 to 6, wherein R₂ is a hydrogen atom, a C₁-C₃ alkyl group or a C₇₋C₁₂ aralkyl group which may have one or more substituents selected from a C₁-C₃ alkyl group, a C₁-C₃ alkoxy group and a halogen atom.

8. The medicament according to any one of Claims 1 to 7, wherein R₂ is a C₇-C₁₂ aralkyl group which may have one or more substituents selected from a hydrogen atom and a C₁-C₃ alkoxy group.

9. The medicament according to any one of Claims 1 to 8, wherein R₂ is a hydrogen atom.

10. The medicament according to any one of Claims 1 to 9, wherein R₁ is a hydrogen atom, a C₁-C₆ alkyl group, a C₅-C₆ cycloalkyl group, a trifluoromethyl group, a halogen atom or a phenyl group.

11. The medicament according to any one of Claims 1 to 10, wherein R₁ is a C₁-C₃ alkyl group, a cyclohexyl group, a trifluoromethyl group, a chlorine atom, a bromine atom or a phenyl group.

12. The medicament according to any one of Claims 1 to 11, wherein R₁ is a methyl group or a propyl group.

13. The medicament according to any one of Claims 1 to 4, wherein the active ingredient is selected from the following compounds:
5-methyl-2-(1-piperazinyl)benzenesulfonic acid;
5-trifluoromethyl-2-(1-piperazinyl)benzenesulfonic acid;
5-n-propyl-2-(1-piperazinyl)benzenesulfonic acid;
5-phenyl-2-(1-piperazinyl)benzenesulfonic acid;
5-chloro-2-(1-piperazinyl)benzenesulfonic acid;
5-bromo-2-(1-piperazinyl)benzenesulfonic acid;
5-iso-propyl-2-(1-piperazinyl)benzenesulfonic acid;
5-cyclohexyl-2-(1-piperazinyl)benzenesulfonic acid;
5-n-propyl-2-(1-homopiperazinyl)benzenesulfonic acid;
5-n-propyl-2-[4-(2,3,4-trimethoxybenzyl)-1-piperazinyl]benzenesulfonic acid; and
5-n-propyl-2-[4-(3,4-dimethoxybenzyl)-1-piperazinyl]benzenesulfonic acid.

14. The medicament according to Claim 13, wherein the active ingredient is selected from the following compounds:
5-methyl-2-(1-piperazinyl)benzenesulfonic acid; and
5-n-propyl-2-(1-piperazinyl)benzenesulfonic acid.

15. The medicament according to any one of Claims 1 to 14, wherein the active ingredient is 5-methyl-2-(1-piperazinyl)benzenesulfonic acid monohydrate.

16. A medicament for preventing and/or treating anterior ST-segment elevation myocardial infarction, which comprises as an active ingredient 5-methyl-2-(1-piperazinyl)benzenesulfonic acid monohydrate, **characterized in that** it is administered to a patient undergoing percutaneous coronary intervention.

17. The medicament according to any one of Claims 1 to 16, **characterized in that** the patient undergoing percutaneous coronary intervention has TIMI flow grade 0 or 1.

18. The medicament according to any one of Claims 1 to 17, **characterized in that** administration of the active ingredient is initiated before percutaneous coronary intervention is performed.

19. The medicament according to any one of Claims 1 to 18, **characterized in that** the active ingredient is continuously administered intravenously for 48 hours.

20. A method for preventing and/or treating an ischemic cardiovascular disease **characterized by** administering a medicament comprising as an active ingredient a compound of an aminobenzenesulfonic acid derivative represented by the following general formula (I): 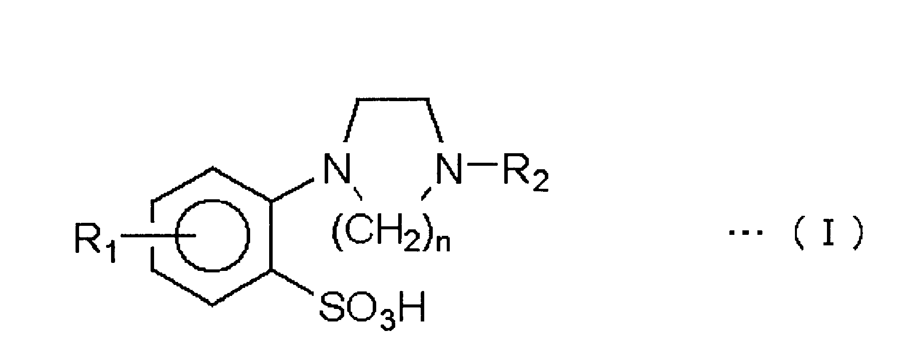 (wherein R₁ represents a hydrogen atom, a C₁-C₆ alkyl group, a C₃-C₇ cycloalkyl group, a halogenated C₁-C₄ alkyl group, a halogen atom or a C₆-C₁₂ aryl group; R₂ represents a hydrogen atom, a C₁-C₆ alkyl group or a C₇-C₁₂ aralkyl group which may have one or more substituents selected from the group consisting of a cyano group, a nitro group, a C₁-C₆ alkoxy group, a halogen atom, a C₁-C₆ alkyl group and an amino group; and n represents an integer of 1 to 4) or a salt thereof, or a hydrate or solvate thereof to a patient undergoing percutaneous coronary intervention.

21. The method according to Claim 20, wherein the ischemic cardiovascular disease is myocardial infarction or angina pectoris.

22. The method according to Claim 21, wherein the myocardial infarction is ST-segment elevation myocardial infarction.

23. The method according to Claim 22, wherein the ST-segment elevation myocardial infarction is anterior ST-segment elevation myocardial infarction.

24. The method according to any one of Claims 20 to 23, wherein the substitution position of R₁ is the 5-position.

25. The method according to any one of Claims 20 to 24, wherein n is 2.

26. The method according to any one of Claims 20 to 25, wherein R₂ is a hydrogen atom, a C₁-C₃ alkyl group or a C₇₋C₁₂ aralkyl group which may have one or more substituents selected from a C₁-C₃ alkyl group, a C₁-C₃ alkoxy group and a halogen atom.

27. The method according to any one of Claims 20 to 26, wherein R₂ is a C₇-C₁₂ aralkyl group which may have one or more substituents selected from a hydrogen atom and a C₁-C₃ alkoxy group.

28. The method according to any one of Claims 20 to 27, wherein R₂ is a hydrogen atom.

29. The method according to any one of Claims 20 to 28, wherein R₁ is a hydrogen atom, a C₁-C₆ alkyl group, a C₅-C₆ cycloalkyl group, a trifluoromethyl group, a halogen atom or a phenyl group.

30. The method according to any one of Claims 20 to 29, wherein R₁ is a C₁-C₃ alkyl group, a cyclohexyl group, a trifluoromethyl group, a chlorine atom, a bromine atom or a phenyl group.

31. The method according to any one of Claims 20 to 30, wherein R₁ is a methyl group or a propyl group.

32. The method according to any one of Claims 20 to 23, wherein the active ingredient is selected from the following compounds:
5-methyl-2-(1-piperazinyl)benzenesulfonic acid;
5-trifluoromethyl-2-(1-piperazinyl)benzenesulfonic acid;
5-n-propyl-2-(1-piperazinyl)benzenesulfonic acid;
5-phenyl-2-(1-piperazinyl)benzenesulfonic acid;
5-chloro-2-(1-piperazinyl)benzenesulfonic acid;
5-bromo-2-(1-piperazinyl)benzenesulfonic acid;
5-iso-propyl-2-(1-piperazinyl)benzenesulfonic acid;
5-cyclohexyl-2-(1-piperazinyl)benzenesulfonic acid;
5-n-propyl-2-(1-homopiperazinyl)benzenesulfonic acid;
5-n-propyl-2-[4-(2,3,4-trimethoxybenzyl)-1-piperazinyl]benzenesulfonic acid; and
5-n-propyl-2-[4-(3,4-dimethoxybenzyl)-1-piperazinyl]benzenesulfonic acid.

33. The method according to Claim 32, wherein the active ingredient is selected from the following compounds:
5-methyl-2-(1-piperazinyl)benzenesulfonic acid; and
5-n-propyl-2-(1-piperazinyl)benzenesulfonic acid.

34. The method according to any one of Claims 20 to 33, wherein the active ingredient is 5-methyl-2-(1-piperazinyl)benzenesulfonic acid monohydrate.

35. A method for preventing and/or treating anterior ST-segment elevation myocardial infarction **characterized by** administering a medicament comprising as an active ingredient 5-methyl-2-(1-piperazinyl)benzenesulfonic acid monohydrate to a patient undergoing percutaneous coronary intervention.

36. The method according to any one of Claims 20 to 35, **characterized in that** the patient undergoing percutaneous coronary intervention has TIMI flow grade 0 or 1.

37. The method according to any one of Claims 20 to 36, **characterized in that** administration of the active ingredient is initiated before percutaneous coronary intervention is performed.

38. The medicament according to any one of Claims 20 to 37, **characterized in that** the active ingredient is continuously administered intravenously for 48 hours.
